# EUROPÄISCHE PATENTANMELDUNG

(11) **EP 2 883 519 A1**
(43) Veröffentlichungstag der Anmeldung: **17.06.2015**
(21) Anmeldenummer: 14197067.3
(22) Anmeldetag: 09.12.2014
(51) Int. Cl.: A61F 2/30, A61F 2/40

(54) **Humeruskopfprothese**

(30) Priorität: 10.12.2013 DE 102013113804
(71) Anmelder: Krankenhaus Buchholz und Winsen gemeinnützige GmbH, 21244 Buchholz (DE)
(72) Erfinder: Hinkenhann, Bernd, Dr., 21224 Rosengarten (DE)
(74) Vertreter: Müller Schupfner & Partner Patent- und Rechtsanwaltspartnerschaft mbB

(57) **Zusammenfassung**

Die Erfindung betrifft eine Humeruskopfprothese für ein Schultergelenk, umfassend einen Lagerkörper, einen Schaft und einen Übergangskörper, der zwischen dem Lagerkörper und dem Schaft angeordnet ist, wobei der Übergangskörper aus einem porösen Material gefertigt ist, das zum Ansatz von Knochenfragmenten, Muskel- und/oder Sehnenansätzen dient und in das vorzugsweise zumindest eine Kavität eingebracht ist.

## Beschreibung

Die vorliegende Erfindung betrifft eine Humeruskopfprothese.

Der obere Teil eines Oberarmknochens (Humerus) gliedert sich in den Humerusschaft und ein proximales Ende. Das proximale Ende umfasst den Humeruskopf (Caput humeri), den Humerushals (Collum anatomicum), den großen Rollhügel (Tuberculum majus) sowie den kleinen Rollhügel (Tuberculum minus). Zwischen den beiden Rollhügeln liegt eine Rollhügelrinne (Sulcus intertubercularis).

Das Schultergelenk, auch Glenohumeralgelenk genannt, wird im Wesentlichen von dem Humeruskopf und der Schulterblattgelenkpfanne (Glenoid) des Schulterblatts (Scapula) gebildet. Die Gelenkfläche der Schulterblattgelenkpfanne ist im Vergleich zum Oberarmknochenkopf klein und umschließt ihn nur zu einem geringeren Teil. Eine Vergrößerung der Kontaktfläche zwischen beiden Gelenkpartnern bildet eine drei bis vier Millimeter breite faserknorpelige Pfannenlippe, die an der Gelenkfläche befestigt ist, die die Kontaktfläche zum Humeruskopf vergrößert und die gleichzeitig Ursprung der langen Bizepssehne und der glenohumeralen Bänder ist. Die beiden Gelenkflächen sind mit einer elastischen, glatten Knorpelschicht überzogen. Eine Gelenkschmiere verhindert, dass beide Knochen stumpf aneinander reiben.

Bei schwerwiegenden Schäden des Schultergelenks ist es häufig angezeigt, eine Schultergelenkprothese einzusetzen. Derartige Schäden können durch Erkrankungen oder durch Verletzungen entstehen. Zu den Erkrankungen gehören unter anderem die Arthrose (Gelenkverschleiß), Gelenkentzündungen wie Gelenk-Rheuma, Entzündungen anderer Ursache, z. B. durch Krankheitserreger wie Bakterien oder Tumore. Eine Erkrankung, die speziell im Schultergelenk vorkommt, ist ein Gewebeuntergang des Oberarmkopfes (Humeruskopfnekrose). Ebenfalls können Verletzungen am Gelenk oder Knochenbrüche Anlass dafür sein, dass ein künstliches Schultergelenk eingepflanzt wird. Eine starke Vorschädigung der Knochensubstanz, z. B. durch Osteoporose, kann die Gefahr eines Knochenbruchs erhöhen. Die Schäden führen in der Schulter zu einer Minderbeweglichkeit und zu Schmerzen. Durch den Ersatz des Schultergelenks oder Teilen davon ist eine Wiedererlangung der Beweglichkeit und eine Besserung der Schmerzen möglich.

Schultergelenkprothesen sind in verschiedenen Varianten bekannt. Ein Oberflächenersatz des Oberarmkopfes besteht lediglich aus einer Metallkappe, die nach entsprechender Vorbereitung auf dem Oberarmkopf befestigt wird. In anderen Fällen wird ein umfassender Ersatz des Oberarmkopfes notwendig. Dazu wird ein Anteil des Knochens herausgenommen und durch eine Oberarmkopfprothese (Humeruskopfprothese) ersetzt, die einen Stiel zur Verankerung besitzt. Oftmals muss darüber hinaus auch die Schultergelenkpfanne bearbeitet und mit einem Pfannenersatz versehen werden. Werden beide Anteile des Gelenks, also Gelenkkopf und Gelenkpfanne, ersetzt, handelt es sich um eine Totalprothese. Beim Ersatz nur einer Gelenkfläche liegt eine Hemiprothese vor.

Bei der Schulterendoprothetik, insbesondere im Falle eines Bruches, entscheidet insbesondere das Anheilen der Knochenbruchstücke an die Prothese über den positiven Verlauf des Eingriffs. Aufgabe der erfindungsgemäß ausgestalteten Humeruskopfprothese ist es, bei weitgehendem Erhalt von Knochensubstanz, die Rekonstruktion des Schultergelenks und damit eine Wiederherstellung der normalen Kinematik zu ermöglichen. Insbesondere ist ein schnelles Anheilen von Knochenfragmenten an der Humeruskopfprothese Aufgabe der vorliegenden Erfindung und es gilt insbesondere ein sicheres und schnelles Anwachsen von Tuberculum majus und minus bzw. deren Fragmenten, an denen die Rotatoren ansetzen, an der Humeruskopfprothese zu ermöglichen.

Obige und weitere Aufgaben werden erfindungsgemäß durch eine Humeruskopfprothese umfassend einen Lagerkörper, einen Schaft und einen Übergangskörper, der zwischen dem Lagerkörper und dem Schaft angeordnet ist, gelöst, wobei der Übergangskörper aus einem porösen Material besteht und an dem Schaftende, das dem Lagerkörper zugewandt ist, und zumindest im metaphysären Bereich angeordnet ist und der Übergangskörper den Schaft zumindest teilweise im Umfang umfasst und wobei das poröse Material aus einem keramischen oder metallischen Material besteht mit offenen Poren in dessen Volumen und mit Porenöffnungen auf dessen Oberfläche, wobei die ein Freivolumen bildenden Poren mehr als 50 Vol.% des Gesamtvolumens des Übergangskörpers ausmachen. Bevorzugte Ausgestaltungen sind Gegenstand der Unteransprüche oder nachfolgend beschrieben.

Die Humeruskopfprothese kann Teil einer Totalprothese oder Hemiprothese sein und umfasst zumindest einen Lagerkörper als Gelenkkörperanteil der Prothese, einem Übergangskörper aus porösem Material und einen Schaft.

Der Schaft der Humeruskopfprothese ist dazu vorgesehen, um in den Oberarmschaft implantiert zu werden. Er sorgt für eine sichere Verbindung von Oberarmknochenrest und Humeruskopfprothese. Dies kann durch Einzementierung des Schafts mit Knochenzement erfolgen. Möglich ist aber auch eine zementfreie Verankerung, wenn der Schaft der Prothese passgenau in den Knochen eingefügt wird.

Der Schaft stellt unmittelbar eine Verbindung zum Lagerkörper oder mittelbar, z. B. über ein Verbindungstück, her, vorzugsweise ein Verbindungstück in Form einer Exzenterscheibe.

Das obere Ende des Schafts ist vom Übergangskörper zumindest teilweise umgeben. Der Übergangskörper umschließt das obere Schaftende zumindest lateral (auf der der Körpermitte abgewandten Seite) und ist aus einem porösen Material gebildet.

Der Übergangskörper schließt vorzugsweise unmittelbar an den Lagerkörper an, insbesondere ohne Ausbildung einer Stufe, und auch unabhängig hiervon besonders bevorzugt mit seiner Außenfläche über zumindest 50%, vorzugsweise mindestens 80% des Umfangs des Lagerkörpers, so dass die Außenfläche der Gelenkfläche entweder in den Übergangskörper oder in den Schaft übergeht (letzteres, wenn der Übergangskörper den Schaft nicht vollständig umgibt) bzw. bei einem inversen Gelenk, die Außenfläche des Übergangskörpers oder des Schaftendes allseitig in die Mantelfläche des Lagerkörpers, die die Gelenkpfanne umschließt, übergeht.

Ebenso ist es möglich, den äußeren Umfang des Übergangskörpers je nach Stellung des aufgesetzten Lagerkörpers anzupassen, um einen gleichmäßigen und nach der Bearbeitung stufenfreien Übergang der Umfangsflächen herzustellen. Hierfür kann z.B. vorgesehen sein, dass die Umfangsfläche des Übergangskörpers stufenförmig die Umfangsfläche des Lagerkörpers übersteht oder die Umfangsfläche des Lagerkörpers abschnittsweise in die Umfangsfläche des Übergangskörpers übergeht und abschnittsweise die Umfangsfläche des Übergangskörpers stufenförmig die Umfangsfläche des Lagerkörpers übersteht. Die Stufe wird dann jeweils, nach oder vor dem Einsetzen des Übergangskörpers, abgetragen, z. B. indem die Stufe mit einer Zange stückweise herausgebrochen wird.

Der Übergangskörper schließt nach nach einer Ausgestaltung unten, vorzugsweise unmittelbar, an den Knochenrest an, insbesondere die Knochenbruchstelle. Etwaige Überstände können vor, aber auch nach dem Einsetzen des Übergangskörpers abgetragen werden, z. B. indem die Überstände mit einer Zange stückweise herausgebrochen werden.

Der Übergangskörper ist mit dem Schaft lösbar verbunden. Je nach Patient können, abgestimmt auf die Größe des Lagerkörpers bzw. dessen Position relativ zum Schaft, unterschiedlich große Übergangskörper bereitgestellt werden, die jeweils mit dem Schaftende verbindbar sind, etwa durch Aufstecken von oben oder vorzugsweise seitliches Aufschieben. Insbesondere sind das Schaftende und die korrespondierende Aufnahme im Übergangskörper normiert, während der Schaft und der Lagerkörper in Größe und Form ansonsten jeweils unterschiedlich, aber untereinander frei mit der Aufnahme im Übergangskörper kombinierbar bereitgestellt werden.

Lagerkörper, Schaft und Übergangskörper liegen somit separat voneinander vor und sind getrennt handhabbar. Vorzugsweise ist das Schaftende und sind die jeweiligen Verbindungsstellen bzw. Aufnahmen am Lagerkörper und Übergangskörper jeweils gleich ausgebildet, so dass verschiedene Schäfte, verschiedene Übergangskörper und verschiedene Lagerkörper jeweils miteinander verbindbar sind. Weiter bevorzugt sind Lagerkörper und Schaft über eine identische (fakultative) Exzenterscheibe verbindbar.

Nach eine bevorzugten Ausführungsform wird der Übergangskörper mit Schrauben an dafür vorgesehenen Gewindebohrungen am Schaft fixiert.

Der Übergangskörper ist zumindest im metaphysären Bereich angeordnet und begünstigt das Zusammenwachsen von Knochenfragmenten, insbesondere solchen mit anhängenden Sehnen der Rotatorenmanschette, mit dem porösen Material und ggf. auch mit dem Knochen des vorhandenen Oberarmschaftes. Der Übergangskörper stellt im metaphysären Bereich Oberfläche für das Zusammenwachsen bzw. Anwachsen von Knochen- und Knorpelfragmenten zur Verfügung. Metaphyse ist der Knochenabschnitt zwischen Diaphyse (Knochenschaft) und Epiphyse.

Insbesondere ergibt sich im Falle einer Fraktur eine Zugänglichkeit von spongiösem Knochenmaterial, z. B. am Tuberculum, das in das poröse Material des Übergangskörpers eindrückbar oder anheftbar ist und dort ggf. unter leichter Deformation des spongiösen Knochenmaterials verankerbar ist. Im Weiteren können auch überschüssige Knochenstücke, insbesondere angrenzend an das Tuberculum, in das poröse Material eingedrückt werden, um das Einwachsen des Knochens in die Prothese zu unterstützen.

Die Knochenfragmente können zusätzlich mit Drähten oder Fäden, z. B. in Form von Cerklagen, an der Prothese verankert werden. Ebenso ist es möglich, die Knochenfragmente mit Hilfe von Schrauben zu verankern, die sich z. B. mit selbstschneidendem Gewinde in das poröse Material einschrauben.

Der Übergangskörper wird genutzt um das ursprüngliche Volumen des Oberarmkopfes im metaphysären Bereich bereitzustellen, wobei im Operationsverlauf oder davor Volumen aus dem Übergangbereich herausgebrochen wird, um Kavitäten zur Aufnahme der einzusetzenden Knochenstücke, insbesondere der Tuberculum-Fragmente, zu schaffen.

Bildgebende Verfahren, wie die Computertomographie, können genutzt werden um sich vor der Operation ein Bild von der Lage und Größe der Kavitäten zu schaffen, zumindest für die Tuberculum-Fragmente. Die Bearbeitung des Übergangskörpers, z. B. mit einer Luer'schen Zange, erfolgt vorzugsweise nach Aufstecken / Aufschieben des Übergangskörpers auf den Schaft, Befestigen an dem Schaft und Einsetzen des Lagerkörpers.

Das poröse Material besteht aus einem keramischen oder metallischen Material, das eine Vielzahl von Poren aufweist, wobei die Poren, die das Freivolumen bilden, mehr als 50 Vol.%, insbesondere mehr als 75 Vol.%, des Gesamtvolumens des Übergangskörpers ausmachen und/oder nach einer anderen Definition die Porenöffnungen mehr als 50 Flächen%, insbesondere mehr als 75 Flächen% der äußeren freien Gesamtoberfläche des Übergangskörper-Materials ausmachen. Die äußere freie Gesamtoberfläche ist die Fläche, die von der Prothese weg nach außen gerichtet ist und nicht vom Schaft, dem Lagerkörper oder dem Verbindungstück (Exzenterscheibe), wenn vorhanden, abgedeckt ist.

Die unmittelbar an die äußere Oberfläche angrenzenden Poren sind vorzugsweise überwiegend (hinsichtlich der Anzahl > 50%, insbesondere größer 80%) offene Poren. Unabhängig hiervon sind die Poren besonders bevorzugt in der Mehrzahl (zu größer 50 Vol.%), insbesondere zu größer 80 Vol.%, bezogen auf das Gesamtvolumen aller Poren, miteinander verbunden und enden offenporig an der Oberfläche. Die Porengröße kann von innen nach außen ansteigen, um an der Oberfläche große offene Poren und eine einfache Bearbeitbarkeit und innen eine hohe Festigkeit aufzuweisen. Die mittlere Porengröße der Porenöffnungen an der Oberfläche liegt vorzugsweise zwischen 0,2 und 5 mm, insbesondere bevorzugt zwischen 0,5 und 1,5 mm.

Das poröse Material ist biokompatibel bzw. bioinaktiv und soll eine positive Interaktion durch Bindung von Knochen entlang der Grenzfläche zum Implantat bewirken.

Geeignete Materialien für das poröse Material sind Metalle, wie rostfreie Stähle, Titanium und titanium-haltige Legierungen, Tantalum und tantalum-haltige Legierungen, Co-Cr-Legierungen, Polymere wie Polyethylen (PE), Polymethylmethacrylat (PMMA) oder Keramiken wie Al₂O₃, ZrO₂ und Calciumphosphate.

Besonders bevorzug sind offenporige Metallschwämme, bestehend aus einem dreidimensionalen Netzwerk miteinander verknüpfter offener Poren, die von fluiden Medien durchströmt werden können. Diese weisen eine sehr große innere Oberfläche auf und ahmen das Konstruktionsprinzip von Naturschwämmen nach. Offenporige Metallschwämme sind z. B. herstellbar nach folgenden Verfahren:
- Feingussverfahren unter Herstellen einer keramischen Gießform ausgehend von einem Wachs- oder Polymerschaumtemplat, ausbrennen des Templats, eingießen einer Metallschmelze und entfernen der keramischen Gießform (z.B. Al, Mg, Ni-Cr, Edelstahl, Cu),
- Gasphasenabscheidung (CVD, PVD) oder Elektrodeposition von Metallen auf einem Polymerschaumprecursor und ausbrennen des Polymerschaums (z.B. Ni, Ti).

Nach dem Feingussverfahren wird z. B. ein Platzhalter, bestehend aus einem offenporigen, mit Wachs stabilisierten Polymerschaum (meist Polyurethan, PUR), mit einem Schlicker aus feuerfestem Material (z. B. Mullit, Gips oder Sand) umgossen und infiltriert. Die Porengröße und Stegdicke des Platzhalters sind für die Eigenschaften des späteren Metallschaums ausschlaggebend. Der feuerfeste Schlicker wird erst getrocknet und anschließend gebrannt. Beim Brennen zersetzt sich der Platzhalter und wird so aus dem Füllstoff entfernt. Die Gießform wird anschließend mit flüssigem Metall ausgegossen. Das Metall nimmt die vormalige Struktur des Platzhalters ein. Nach dem Erstarren des Metalls wird der Füllstoff im letzten Schritt beispielsweise mit unter Hochdruck stehendem Wasser entfernt. Nach diesem Verfahren können Bauteilgeometrie, Dichte und Zellstruktur gezielt hergestellt werden. Es können sehr regelmäßige Strukturen und Porengrößenverteilungen erzeugt werden. Feste Bauteile, wie Hülsen für die Schraubverbindung oder eine Hülse zum Aufstecken auf den Schaft, können direkt beim Gießen des Metallschwammes an den Schaum angebunden werden.

Ein schwammartiger, aus Tantal oder Titan bestehender Werkstoff ist z. B. in einem Hochtemperatur-Vakuumverfahren durch Gasphasenabscheidung von Tantalpentachlorid oder Titantetrachlorid auf einem retikulierten Polyurethanschaumstoff erhältlich. Es scheidet sich Tantal bzw. Titan auf dem Schaumstoff ab und gebildetes Chlorgas wird abgeführt.

Tantal-Metallschwämme sind z. B. von der Firma Zimmer Holdings Inc., Warsaw, Indiana, USA unter dem Namen "Trabecular Metal" oder unter dem Namen Regenerex® von der Biomet, Inc., Warsaw, Indiana, USA erhältlich.

Der Schaft kann auch nur mittelbar über ein Verbindungstück, z. B. in Form einer Exzenterscheibe, mit dem Lagerkörper in Verbindung stehen. Insbesondere sind Lagerkörper und Schaft so ausgebildet, dass jeweils unterschiedliche Lagerkörper und unterschiedliche Schafte ausgewählt anhand bildgebender Verfahren, die die Schulteranatomie des Patienten ermitteln, über ein einheitliches Verbindungsstück verknüpfbar sind. Das Verbindungsstück ist vorzugsweise eine Exzenterscheibe mit Befestigungsmitteln, insbesondere in Form von sich konisch verjüngenden Stiften, für Lagerkörper und Schafte aufweisend zwei versetzte Drehachsen. Vorzugsweis findet das Verbindungstück und insbesondere die Exzenterscheibe Aufnahme im Lagerkörper und wird vom Lagerkörper insbesondere vollumfänglich aufgenommen, abgesehen von der Seite, die auf dem Schaft aufliegt und dem Befestigungsmittel für den Schaft, wenn es sich um einen Stift und eine Aufnahme für den Stift im Schaft handelt.

Der Lagerkörper bildet den Gelenkkopf von halbkugelähnlicher Form aus zur Anlage an die Gelenkpfanne. In Richtung auf den Schaft ist der Lagerkörper vorzugsweise im Wesentlichen plan ausgebildet, abgesehen ggf. von der Ausnehmung zur Aufnahme der Exzenterscheibe. Der Lagerkörper kann bei Verwendung einer Prothese mit inversem Gelenk unter Substitution der natürlichen Gelenkpfanne auch als Pfanne ausgebildet sein.

Lagerkörper, Verbindungsstück und Schaft sind z. B. aus Chrom-Nickel-Molybdän oder Titanium ausgebildet.

Soweit gewünscht können Hilfsstoffe in das poröse Material des Übergangskörpers eingebracht oder auf dieses aufgezogen werden. Geeignete Hilfsstoffe sind z.B. das Knochenwachstum stimulierende Substanzen, Angiogenesestimulierenden Faktoren, antibakterielle Mittel und Entzündungshemmer. Um die Biokompatibilität des Mantel-Struktur zu verbessern, können die Poren mit Substanzen die Calcium und / oder Phosphat enthalten vorsehen sein. Beispiele hierfür sind Hydroxyapatit, Fluorapatit, Tricalziumphosphat und Tetracalciumphosphat, Octacalciumphosphat, Brushit oder Calciumcarbonat.

Das Verfahren der Einbringung der Humeruskopfprothese kann wie folgt beschrieben werden:

Nach operativer Freilegung des Schultergelenkes werden zunächst die abgebrochenen Fragmente des Oberarmkopfes voneinander gelöst, denn sie hängen mit ihren anhaftenden Sehen noch aneinander. Dann wird der Schaft mit Raspeln in aufsteigender Größe aufgeraspelt und ein Probe-Schaft eingebracht der der zuletzt verwendeten Raspelgröße entspricht. Nun werden Probe-Exzenterscheibe und Probe-Lagerkörper aufgesetzt.

Bei der Probe-Prothese sind Exzenterscheibe und Lagerkörper leicht gegeneinander verdrehbar und über Markierungen zur Position und Ausrichtung wird die tatsächliche Prothese ausgerichtet. Nachfolgend wird die Probe-Prothese aus dem Oberarmknochen herausgenommen und dient als Schablone für die Orginal-Prothese, nachfolgend kurz Prothese.

Die Prothese wird exkorporal zusammensetzt indem die Exzenterscheibe mit ihrem unteren Stift in die korrespondierende Ausnehmung im Schaft eingelassen und der Lagerkörper auf die Exzenterscheibe aufgesetzt wird, so dass die Exzenterscheibe in der Aushöhlung des Lagerkörpers verschwindet. Gleichzeitig greift der obere Stift in die Ausnehmung innerhalb der Aushöhlung des Lagerkörpers ein. Anhand der Probe-Prothese erfolgt nun eine Ausrichtung von Schaft, Exzenterscheibe und Lagerkörper. Schaft, Exzenterscheibe und Lagerkörper werden dann mit einer Zwinge verpresst, womit die Stifte jeweils einen sicheren unverrückbaren Halt in ihren jeweiligen Ausnehmungen erhalten. Nun wird der Schaft in den aufgeraspelten Oberarmknochen eingesetzt und kann - bei gutem Knochen - zementfrei durch Verklemmung, oder - bei schlechtem Knochen - zementiert fixiert werden.

Es wird ein Übergangskörper passender Größe ausgewählt und seitlich auf den in den Knochen eingesetzten Schaft aufgeschoben, dort wo der Schaft aus dem Knochen heraussteht. Es werden zwei Schrauben durch den Übergangskörper geschoben und in Gewindebohrungen des Schafts durch Eindrehen fixiert. Im Idealfall reicht der Übergangskörper sowohl von der Bruchstelle des Knochens bis zum Lagerkörper als auch an die äußere Umfangslinie des Lagerkörpers heran, wobei etwaige Überstände mit Hilfe einer Zange herausgebrochen werden bzw. am anderen Ende der Übergangskörper vor dem Aufschieben auf die richtige Länge gebracht ist und etwaige Überstände im eingesetzten Zustand ebenso mit der Zange herausgebrochen werden.

Nun werden aus dem offenporigen Metall des Übergangskörpers mit der Zange Kavitäten geschaffen, die exakt der Größe der abgebrochenen Fragmente des Oberarmkopfes entsprechen. In diese Kavitäten werden die Fragmente mit ihren anhängenden Sehnen der Rotatorenmanschette so passgenau wie möglich eingebracht und mit Schrauben, Stiften und/oder Drahtschlingen fixiert. So ist eine sichere Verankerung der Fragmente mit ihren anhängenden Sehnen gewährleistet und die Rotatorenmanschette auf dem Lagerkörper aufgespannt. Dabei wird darauf geachtet, dass die Kavitäten nicht nur die korrekte Größe, sondern auch die korrekte Position im Raum erhalten, damit die Sehnen ihre anatomische Zugrichtung behalten. Die Operation kann sodann durch Wundverschluss beendet werden.

Die Erfindung wird nachfolgend unter Bezugnahme auf die Zeichnungen erläutert, ohne auf die Ausführungsform gemäß Zeichnungen beschränkt zu sein. Es zeigen:

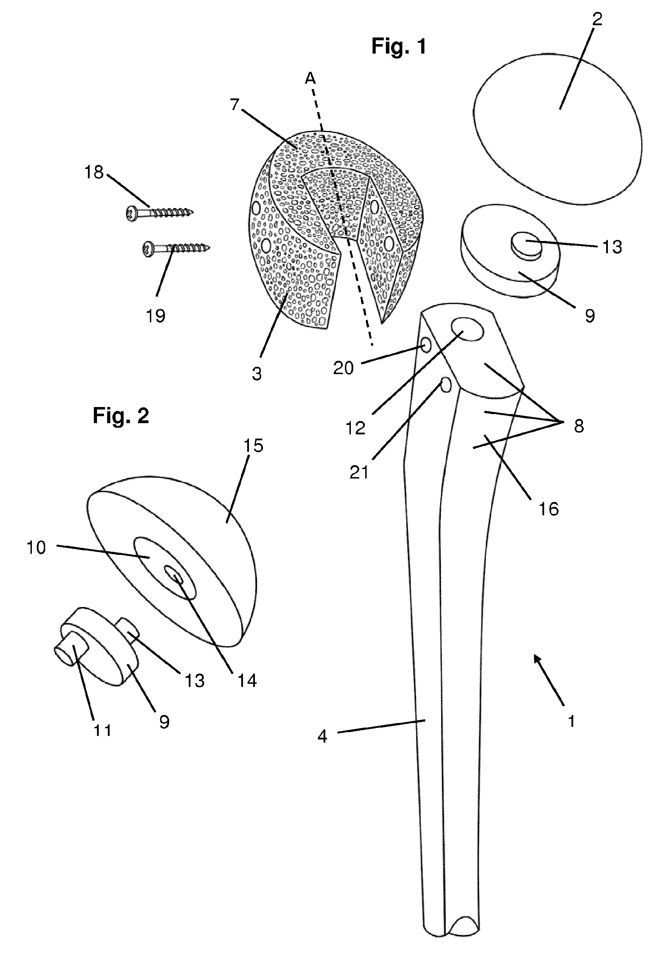

Fig. 1 zeigt die Humeruskopfprothese in einer Explosionsansicht.
Fig. 2 zeigt Exzenterscheibe und Lagerkörper der Explosionsansicht von schräg unten.

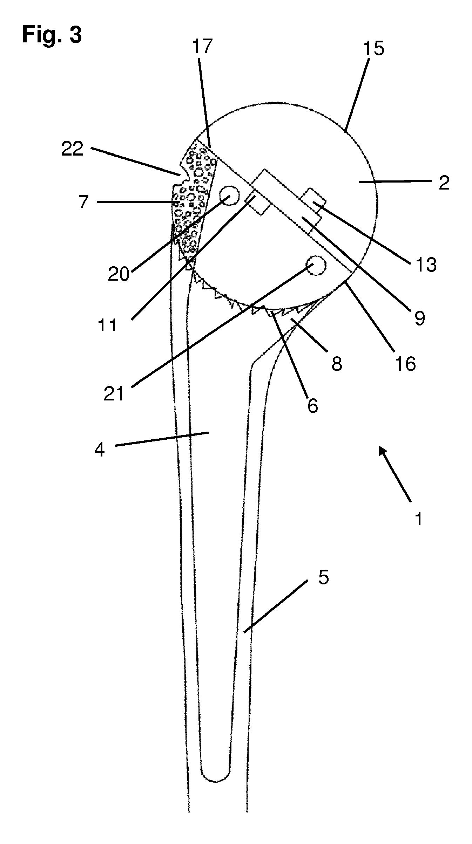

Fig. 3 zeigt in einer schematischen Darstellung einen mittigen Schnitt durch einen rechten Humerusknochen mit eingesetzter Humeruskopfprothese längs einer gedachten Fläche parallel zur Frontalebene.

Die Humeruskopfprothese 1 umfasst einen Lagerkörper 2 als Glenkkörperanteil, einen Übergangskörper 3 aus porösem Material und einen Schaft 4. Der Schaft 4 ist in den Oberarmknochen 5 passgenau eingefügt. Unmittelbar an die Knochenbruchstelle 6 schließt sich der Übergangskörper 3 an, der eine Vielzahl von offenen Poren 7 auf der Oberfläche aufweist. Auf das Schaftende 8 ist eine Exzenterscheibe 9 aufgesetzt, die eine Verbindung zum Lagerkörper 2 herstellt. Die Exzenterscheibe 9 findet vollständig Aufnahme in einer Aushöhlung 10 des Lagerkörpers.

Unmittelbar an die Knochenbruchstelle 6 schließt sich der Übergangskörper 3 an, der eine Vielzahl von offenen, durchgängigen Poren 7 auf der Oberfläche und im Inneren aufweist. Hierzu greift ein Stift 11 der Exzenterscheibe 9 in eine Ausnehmung 12 am Ende des Schaftes ein und der andere Stift 13 der Exzenterscheibe 9 in eine Ausnehmung 14 in der Aushöhlung 10 des Lagerkörpers 2 am Schaftende 8. Die Exzenterscheibe 9 findet vollständig und flächenbündig Aufnahme in einer Aushöhlung 10 des Lagerkörpers 2.

Der Lagerkörper ist als Gelenkkopf 15 oben im Wesentlichen als Kugelteilabschnitt (Kugelkalotte) ausgebildet.

Das obere Ende des Schafts ist vom Übergangskörper 3 fast vollständig umschlossen, ausgenommen von einem, zur Körpermitte gerichteten Steg 16, der durch den Schaft gebildet wird. Der Übergangskörper 3 schließt unmittelbar an den Lagerkörper 2 an und umschließt dessen äußeren Umfang 17, so dass die Außenfläche der Gelenkfläche im Wesentlichen allseitig und bündig in den Übergangskörper 3 übergeht (bis auf den Steg 16). Der Übergangskörper 3 schließt nach unten unmittelbar an die Knochenbruchstelle 6 an.

Der Übergangskörper 3 ist mit dem Schaft lösbar verbunden und wird auf diesen seitlich aufgeschoben. Der Übergangskörper ist mit zwei Schrauben 18,19 an dafür vorgesehenen Gewindebohrungen 20, 21 am Schaft fixiert. Der Übergangskörper 3 stellt im metaphysären Bereich Oberfläche für das Zusammenwachsen bzw. Anwachsen von Knochen- und Knorpelfragmenten zur Verfügung.

Weiterhin gezeigt ist ein mit einer Handzange, wie einer Luer'schen Zange, herausgebrochenes Stück aus dem Übergangskörper 3 zur Bildung einer Kavität 22, in die Knochenfragmente des Tuberculum majus bzw. minus eingesetzt und dort verankert werden.

### Bezugszeichenliste

| | |
|---|---|
| Humeruskopfprothese | 1 |
| Lagerkörper | 2 |
| Übergangskörper | 3 |
| Schaft | 4 |
| Oberarmknochen | 5 |
| Knochenbruchstelle | 6 |
| offene Poren | 7 |
| Schaftende | 8 |
| Exzenterscheibe | 9 |
| Aushöhlung des Lagerkörpers | 10 |
| unterer Stift der Exzenterscheibe | 11 |
| Ausnehmung am Ende des Schaftes | 12 |
| Oberer Stift der Exzenterscheibe | 13 |
| Ausnehmung in der Aushöhlung | 14 |
| Gelenkkopf als Kugelteilabschnitt | 15 |
| Steg | 16 |
| äußerer Umfang des Lagerkörpers | 17 |
| Schrauben | 18, 19 |
| Gewindebohrungen | 20, 21 |
| herausgebrochene Kavität | 22 |

## Patentansprüche

1. Humeruskopfprothese (1) umfassend einen Lagerkörper (2), einen Schaft (4) und einen Übergangskörper (3), der zwischen dem Lagerkörper (2) und dem Schaft (4) angeordnet ist,
wobei der Übergangskörper (3) aus einem porösen Material besteht und an dem Schaftende (8), das dem Lagerkörper (2) zugewandt ist, und zumindest im metaphysären Bereich angeordnet ist und der Übergangskörper (3) den Schaft (4) zumindest teilweise im Umfang umfasst,
wobei das poröse Material aus einem keramischen oder metallischen Material besteht mit offenen Poren (7) in dessen Volumen und mit Porenöffnungen auf dessen Oberfläche, wobei die ein Freivolumen bildenden Poren (7) mehr als 50 Vol.% des Gesamtvolumens des Übergangskörpers ausmachen.

2. Humeruskopfprothese nach Anspruch 1, wobei der Lagerkörper (2) und der Schaft (4) über ein Verbindungstück miteinander verbunden sind, insbesondere über eine Exzenterscheibe (9), so dass der Lagerkörper (2) und der Schaft (4) um versetzte Achsen der Exzenterscheibe (9) beweglich miteinander verbunden sind.

3. Humeruskopfprothese nach zumindest einem der vorstehenden Ansprüche, wobei die Poren (7) des porösen Materials auf der Oberfläche des Übergangskörpers (3) im Mittel Durchmesser von 0.2 mm bis 5 mm, vorzugsweise 0,5 bis 1,5 mm, aufweisen.

4. Humeruskopfprothese nach zumindest einem der vorstehenden Ansprüche, **dadurch gekennzeichnet, dass** der Lagerkörper (2) und der Übergangskörper (3) trennbar miteinander verbunden sind.

5. Humeruskopfprothese nach zumindest einem der vorstehenden Ansprüche, wobei das poröse Material ein Metallschwamm aus einer Titanium-Cobalt-Chrom-Legierung, aus Titanium oder aus Tantal ist.

6. Humeruskopfprothese nach zumindest einem der vorstehenden Ansprüche, wobei der Übergangskörper (3) eine oder mehrere Kavitäten (22) zur Aufnahme von einzusetzenden Knochenstücken, insbesondere zur Aufnahme zumindest eines Tuberculum-Fragments aufweist.

7. Humeruskopfprothese nach zumindest einem der vorstehenden Ansprüche, wobei in das poröse Material des Übergangskörpers (3) zumindest eine Kavität (22) mit einer Handzange herausgebrochen ist, vorzugsweise zur Aufnahme. zumindest eines Tuberculumfragments.

8. Humeruskopfprothese nach zumindest einem der vorstehenden Ansprüche, wobei die Poren (7) des porösen Materials Teil eines dreidimensionalen Netzwerks sind und miteinander verknüpfte offene Poren (7) sind, die von fluiden Medien durchströmbar sind.

9. Humeruskopfprothese nach zumindest einem der vorstehenden Ansprüche, wobei die Poren (7) des porösen Materials des Übergangskörpers (3) zu größer 50 Vol.%, vorzugsweise zu größer 80 Vol.%, bezogen auf das Gesamtvolumen aller Poren (7), miteinander verbundene Poren (7) sind, die offenporig an der Oberfläche enden.

10. Humeruskopfprothese nach zumindest einem der vorstehenden Ansprüche, wobei der Lagerkörper (2) ein Kugelkopf (15) ist und im Wesentlichen die Form einer Kugelkalotte hat, wie sie sich als Schnitt einer Ebene mit einem kugelförmigen Körper bildet.

11. Humeruskopfprothese nach zumindest einem der vorstehenden Ansprüche, wobei der Übergangskörper (3) an die Knochenbruchstelle (6) heranreicht, vorzugsweise ohne dass der jeweilige Übergang eine Stufe ausbildet.

12. Humeruskopfprothese nach zumindest einem der vorstehenden Ansprüche, wobei der Übergangskörper (3) an den Lagerkörper (2) heranreicht, zum Beispiel ohne dass der jeweilige Übergang eine Stufe ausbildet.

13. Humeruskopfprothese nach zumindest einem der Ansprüche 1 bis 11, wobei der äußere Umfang des Übergangskörpers an die jeweilige Stellung des aufgesetzten Lagerkörpers angepasst ist, um einen im wesentlichen gleichmäßigen und nach dem Abtragen im wesentlichen stufenfreien Übergang der Umfangsflächen herzustellen, wobei vorgesehen ist, dass zunächst zumindest abschnittsweise der Übergangskörpers die Umfangsfläche des Lagerkörpers stufenförmig übersteht, wobei die Stufe(n) jeweils nach oder vor dem Verbinden des Übergangskörpers mit dem Schaft, insbesondere durch Aufstecken oder Aufschieben, abgetragen wird/werden, vorzugsweise indem die Stufe(n) mit einer Zange stückweise herausgebrochen wird/werden.

14. Humeruskopfprothese nach zumindest einem der vorstehenden Ansprüche, wobei der Übergangskörper mit dem Schaft lösbar verbunden ist und mit dem Schaftende durch Aufstecken von oben oder vorzugsweise seitliches Aufschieben verbindbar ist und vorzugsweise mittels Schrauben durch den Übergangskörper am Schaft fixierbar ist.
